(19) Europäisches Patentamt
European Patent Office
Office européen des brevets

(11) **EP 1 923 379 B1**

(12) **EUROPÄISCHE PATENTSCHRIFT**

(45) Veröffentlichungstag und Bekanntmachung des Hinweises auf die Patenterteilung:
**03.03.2010  Patentblatt 2010/09**

(21) Anmeldenummer: 07120030.7

(22) Anmeldetag: **06.11.2007**

(51) Int Cl.:
*C07B 39/00* (2006.01)     *C07C 45/63* (2006.01)
*C07C 49/14* (2006.01)     *C07C 49/16* (2006.01)
*C07C 49/457* (2006.01)    *C07C 67/307* (2006.01)
*C07C 69/63* (2006.01)     *C07C 69/716* (2006.01)
*C07C 231/12* (2006.01)    *C07C 235/80* (2006.01)

(54) **Verfahren zur Herstellung von chlorierten Carbonylverbindungen in Jet Loop Reaktoren**

Process for preparing chlorinated carbonyl compounds in jet loop reactors

Procédé de préparation de composés carbonylés chlorés dans des réacteurs jet loop

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **13.11.2006  DE 102006053380**

(43) Veröffentlichungstag der Anmeldung:
**21.05.2008  Patentblatt 2008/21**

(73) Patentinhaber: **Wacker Chemie AG**
**81737 München (DE)**

(72) Erfinder:
• **Kutschera, Dirk**
**84524, Neuötting (DE)**
• **Riener, Franz-Xaver**
**84375, Kirchdorf (DE)**
• **Schlüter, Michael**
**28870, Ottersberg (DE)**

(74) Vertreter: **Fränkel, Robert et al**
**Wacker Chemie AG**
**Zentralbereich Patente, Marken und Lizenzen**
**Hanns-Seidel-Platz 4**
**81737 München (DE)**

(56) Entgegenhaltungen:
**EP-A- 0 234 503      DE-A1- 10 010 594**
**DE-C1- 10 223 649**

• **VAN DIERENDONCK, L. L.; ZAHRADNIK, J.; LINEK, V.: "Loop Venturi Reactor-A Feasible Alternative to Stirred Tank Reactors?" INDUSTRIAL & ENGINEERING CHEMISTRY RESEARCH., Bd. 37, 1998, Seiten 734-738, XP002467780 AMERICAN CHEMICAL SOCIETY. WASHINGTON**
• **PETER ZEHNER AND MATTHIAS KRAUME: "Ullmann's Encyclopedia of Industrial Chemistry - Bubble Columns" [Online] 15. Juni 2000 (2000-06-15), WILEY-VCH VERLAG GMBH & CO. KGAA , WEINHEIM , XP002467781 Gefunden im Internet: URL:http: //www.mrw.interscience.wiley.com/ emrw/ 9783527306732/ueic/article/b04_275/cu rrent/pdf DOI: 10.1002/14356007.b04_275> * Seite 22 - Seite 26 ***

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von chlorierten Carbonylverbindungen mit verbesserter Selektivität und verbessertem Umsatz in Jet Loop Reaktoren, wobei die entsprechenden unchlorierten oder teilchlorierten Carbonylverbindungen mit Chlor unter kontinuierlicher oder halbkontinuierlicher Fahrweise zur Reaktion gebracht werden.

Chlorierte Carbonylverbindungen sind Bausteine für zahlreiche Produkte in der synthetischen organischen Chemie für Anwendungen beispielsweise in der Pharma- und Agroindustrie.

In der Literatur werden verschiedene Möglichkeiten beschrieben, Carbonylverbindungen zu chlorieren. Die Chlorierung wird dabei im Rührkessel, Rohrreaktor, einer Kombination von Rohrreaktor und Rührkessel oder in einer Blasensäule durchgeführt. Durch die unzureichende Durchmischung in den einzelnen Reaktortypen kommt es dabei oftmals lokal zu einer inhomogenen Chlorkonzentration und zu Temperaturinhomogenitäten innerhalb des Reaktors, die ein breites Spektrum an Nebenprodukten entstehen lassen. Daraus ergibt sich eine mangelhafte Selektivität und ein mangelhafter Umsatz bezüglich des Zielprodukts.

Die einzelnen, aus dem Stand der Technik bekannten Reaktortypen sind hinsichtlich ihrer Mischleistung weitgehend optimiert. Neben dem Einsatz von Begasungsrührern in Rührkesseln und statischen Mischern in Rohreaktoren werden die unterschiedlichsten Gasverteiler bei Blasensäulen eingesetzt oder aufwändige Chlorierungen in der Gasphase durchgeführt. Die Wärmeabfuhr erfolgt dabei durch interne und gegebenenfalls durch externe Wärmetauscher.

Bei der Chlorierung von Carbonylverbindungen sind die Umsätze oft unvollständig, Selektivitäten und damit Ausbeuten sind oft niedrig. Die dadurch notwendige anschließende Aufarbeitung ist technisch aufwändig, ergibt Reststoffe und verteuert dadurch die Produktion. Zusätzlich sind die chlorierten und teilchlorierten Carbonylverbindungen oft thermisch instabil und neigen zur thermischen Trennung.

Um die Selektivitäten und die Umsätze bei der Chlorierungsreaktion zu steigern, werden im Stand der Technik verschiedene Verfahren vorgeschlagen.

EP0234503 beschreibt beispielsweise die Zugabe von Katalysatoren zur Reaktionsmischung. Beim Einsatz von Katalysatoren ist die für eine hohe Katalysatoraktivität relevante Reinheit der unchlorierten oder teilchlorierten Carbonylverbindungen oftmals nur durch zusätzliche Aufarbeitungsschritte erreichbar und der hohe Katalysatorpreis schlägt sich auf die Herstellungskosten nieder.

GB2036016 beschreibt die Durchführung der Chlorierung in der Gasphase. Diese Verfahren sind technisch sehr aufwändig, da sie zum Teil zu Beginn hohe Reaktionstemperaturen für die Chlorierung in der Gasphase benötigen und durch die Gasphasenreaktion nur eine niedrige Raum-Zeit-Ausbeute erreicht wird. Ebenso sind mehrstufige Aufarbeitungsprozesse für die Edukte und Produkte notwendig, was wiederum Ausbeuteverluste mit sich bringt.

Aus DE19842332 ist ein Jet Loop Reaktor und ein Verfahren und eine Vorrichtung zur biologischen Reinigung von Abwasser bekannt. Ein Hinweis zu Verwendung derartiger Vorrichtungen zur Chlorierung von Verbindungen wird nicht gegeben.

DE 10010594 beschreibt ein spezielles Verfahren zur Herstellung von Chlorcarbonsäurechloriden durch Chlorierung von Lactonen in Anwesenheit einer Borverbindung und eines Chlorierungskatalysators. Die Umsetzung erfolgt in einem Rührkessel. Es wird darauf hingewiesen, dass bei dieser speziellen Reaktion eine ausreichende Durchmischung des Eduktes, des Katalysators der Borverbindung und des Chlorierungsmittels wichtig sei. Dazu werden exemplarisch bekannte Apparate aufgezählt. DE 10010594 nennt dabei unter vielen anderen auch den möglichen Einsatz von Schlaufenreaktoren, ohne näher darauf einzugehen oder diesen in den Beispielen zu erläutern. Dem Fachmann wird kein Hinweis gegeben, den Schlaufenreaktor allgemein für Chlorierungsreaktionen von Carbonylvebindungen zu einzusetzen.

In Van Dierendonck et. al., Industrial Engineering Chemistry Research, Bd. 37, 1998, Seiten 734-738 ("Loop-Venturi-Reactor") werden eine Vielzahl von verschiedenen Reaktionsapparaturen beschrieben. Dabei werden unter anderem auch Jet-Loop Reaktoren als vorteilhafte Alternative zu Rührkesselreaktoren genannt. Auch die Möglichkeit der Chlorierung als eine von vielen Reaktionen wird allgemein genannt, ohne jedoch dem Fachmann Hinweise auf spezielle Ausführungsformen oder Reaktionsparameter zu gegeben. In der Zusammenfassung des Dokuments wird darauf hingewiesen, dass die Reaktorsysteme in Übereinstimmung mit der Literatur, wirtschaftlich besonders attraktiv für Reaktionen oberhalb von 10 bar sind.

Aufgabe der vorliegenden Erfindung ist es, ein Verfahren zur Herstellung von chlorierten oder teilchlorierten Carbonylverbindungen bereitzustellen, welches die Nachteile des Standes der Technik überwindet und ein kostengünstiges Verfahren mit hoher Selektivität und Produktreinheit bereitstellt.

Gegenstand der Erfindung ist ein Verfahren zur Herstellung von chlorierten oder teilchlorierten Carbonylverbindungen, dadurch gekennzeichnet, dass unchlorierte oder teilchlorierte Carbonylverbindungen mit einem Chlorierungsmittel in einem Jet Loop Reaktor zur Reaktion gebracht werden.

Das Prinzip und der Aufbau von Jet Loop Reaktoren ist zusammenfassend in der Literatur beschrieben (Zehner, P.: Bubble Columns 4. Jet Loop Reactors, Ullmann's Encyclopedia of Industrial Chemistry, Article Online Posting Date 15.06.2000). Man unterscheidet zwischen Mammutschlaufenreaktor (Airliftreaktor), Propeller- und Strahlschlaufenreaktor.

Zur Veranschaulichung sind die wichtigsten Typen der verschiedenen Jet Loop Reaktoren in den Figuren 1 bis 5 dargestellt. Figur 1 zeigt die schematische Abbildung eines Strahlzonen-Schlaufenreaktors (SZR) mit Teilrückführung,

Figur 2 die schematische Abbildung eines Strahlzonen-Schlaufenreaktors (SZR) ohne Teilrückführung,

Figur 3 einen Strahlschlaufenreaktor (SSR) mit Teilrückführung,

Figur 4 Strahlschlaufenreaktor (SSR) mit Teilrückführung und Lösungsmitteleinspeisung und Figur 5 Strahlschlaufenreaktor (SSR) ohne Teilrückführung.

Bei den Strahlschlaufenreaktoren, zu denen Kompakt-, Prallstrahl- sowie Treibstrahlschlaufenreaktoren zählen, werden die Flüssigkeit und das Gas durch eine Düse von oben bzw. unten in den Reaktor gefördert, was nicht nur zu einer Umwälzung des Gemischs und somit zu einer Schlaufenbildung, sondern auch zu einer Dispergierung der Gasphase führt.

Bei dieser Art von Treibstrahlreaktoren ist bei hohen Gasbelastungen ein hoher Energieeintrag notwendig, um eine Dispergierung der Gasphase und die Umwälzung des Gemisches zu gewährleisten. Durch den Strahlzonen-Schlaufenreaktor kann der Energiebedarf reduziert werden, da eine Trennung von Dispergierung und Gemischumwälzung erfolgt.

Ein wesentlicher Vorteil von Jet Loop Reaktoren bei der Chlorierungs-Reaktion gegenüber bisher eingesetzten Blasensäulenreaktoren und anderen Gas-Flüssigreaktoren ist die intensivere und schnelle Flüssigkeitsvermischung bei hoher Umlaufströmung und somit ein erhöhter Wärme- und Stoffübergang. Ein weiterer Vorteil ergibt sich bei strahlgetriebenen Schlaufenreaktoren (Strahlschlaufenreaktor = SSR) durch eine feinere Dispergierung der Gasphase und somit einer größeren spezifischen Phasengrenzfläche.

Bevorzugt wird die erfindungsgemäße Reaktion in einem Strahlzonen-Schlaufenreaktor durchgeführt.

Das erfindungsgemäße Verfahren ermöglicht die Herstellung von chlorierten oder teilchlorierten Carbonylverbindung mit hohen Selektivitäten und Umsätzen, ohne technisch aufwändige Verfahrensschritte. In diesem Zusammenhang werden unter chlorierten Carbonylverbindungen solche verstanden, bei denen neben der Carbonylverbindung alle Wasserstoffatome durch Chlor substituiert sind. Teilchlorierte Carbonylverbindungen sind solche, bei denen neben der Carbonylverbindung mindestens ein Wasserstoffatom durch Chlor substituiert ist.

Für die Chlorierung können ein oder mehrere Chlorierungsmittel aus der Gruppe enthaltend Chlor, Sulfurylchlorid und Thionylchlorid verwendet werden. Besonders bevorzugt wird als Chlorierungsmittel Chlor.

Die einsetzbaren unchlorierten oder teilchlorierten Carbonylverbindungen können geradekettig, verzweigt oder von cyclischer Struktur sein.

Die Reste R1 und R2 am Kohlenstoffatom der Carbonylgruppe können gleich oder verschieden sein. Es spielt dabei keine Rolle, ob die Reste bereits ein oder mehrere Halogenatome tragen oder nicht. Teilchlorierte Verbindungen sind solche Verbindungen, bei denen neben der Carbonylverbindung mindestens ein Wasserstoffatom durch Chlor substituiert ist. Die im folgenden Absatz aufgezählten Gruppen können daher statt der dort angegebenen Wasserstoffatome auch ein oder mehrere Chloratome tragen, wo dies chemisch möglich ist.

Beispiele für Carbonylverbindungen, die im erfindungsgemäßen Verfahren eingesetzt werden können, sind Ketone, wobei R1 und R2 verschieden oder gleich sein können und aus der Gruppe enthaltend $-CH_3$ und $-(CH_2)_n-CH_3$ gewählt werden; Cycloketone, wobei R1 und R2 gleich ist und $-(CH_2)_n-$ bedeutet; Aldehyde, wobei R1 für ein Wasserstoffatom steht und R2 die Bedeutung $-(CH_2)_n-CH_3$ hat; 1,3 Dicarbonsäuren, wobei R1 $-O-CH_2-CH_3$ und R2 $-CH_2-CO-O-CH_2-CH_3$ bedeutet; β-Dicarbonylverbindungen, wobei $R_1$ gleich $-(CH_2)_n-CH_3$ ist und R2 aus der der Gruppe enthaltend $-CH_2-CO-CH_3$, $-CH_2-CO-O-(CH_2)n-CH_3$ und $-CH_2-CO-N-R'-R''$ gewählt wird, wobei R' aus der der Gruppe enthaltend H, $CH_3$, $(CH_2)_n$ gewählt wird und R'' aus der der Gruppe enthaltend H, $CH_3$, $(CH_2)_n$ gewählt wird.

Bevorzugte Verbindungen für Ketone sind Monochloraceton, für Cycloketone Cyclopentanon, für Aldehyde Propionaldehyd, für 1,3 Dicarbonsäuren Diethylmalonsäure und für β-Dicarbonylverbindungen Acetylaceton, 2-Acetoacetamid und Acetessigsäureethylester.

Die Chlorzugabe kann dabei unter Normal- oder Überdruck stattfinden, bevorzugt wird bei Normaldruck gearbeitet. Die Reaktion kann in kontinuierlicher oder halbkontinuierlicher Fahrweise stattfinden. Eine kontinuierliche Chlorzugabe kann auch unter Rückführung von nicht umgesetztem Chlor in den Kreislauf erfolgen.

Die Dosierung des Chlorierungsmittels zur unchlorierten oder teilchlorierten Carbonylverbindung in den Reaktor geschieht in einem Volumenstromverhältnis von 1 : 1 bis 1 : 4000, vorzugsweise 1:30 bis 1:2000. Die Dosierung erfolgt dabei über eine Ein- oder Mehrkomponentenmischdüse.

Die Reaktion im Jet Loop Reaktor kann bei Temperaturen von -20°C bis 160°C, bevorzugt bei 5°C bis 100°C, durchgeführt werden.

Die unchlorierten oder teilchlorierten Carbonylverbindungen können bei dem erfindungsgemäßen Verfahren in Substanz oder in Gegenwart eines Lösungsmittel vorliegen. Als Lösungsmittel können alle dem Fachmann bekannten verwendet werden, sofern diese für die jeweils eingesetzte Carbonylverbindung und das Chlorierungsmittel geeignet sind.

Optional kann die Reaktion auch in Anwesenheit eines Katalysators durchgeführt werden. Als Katalysatoren können dabei die dem Fachmann bekannten Katalysatoren aus anderen Chlorierungsreaktionen verwendet werden.

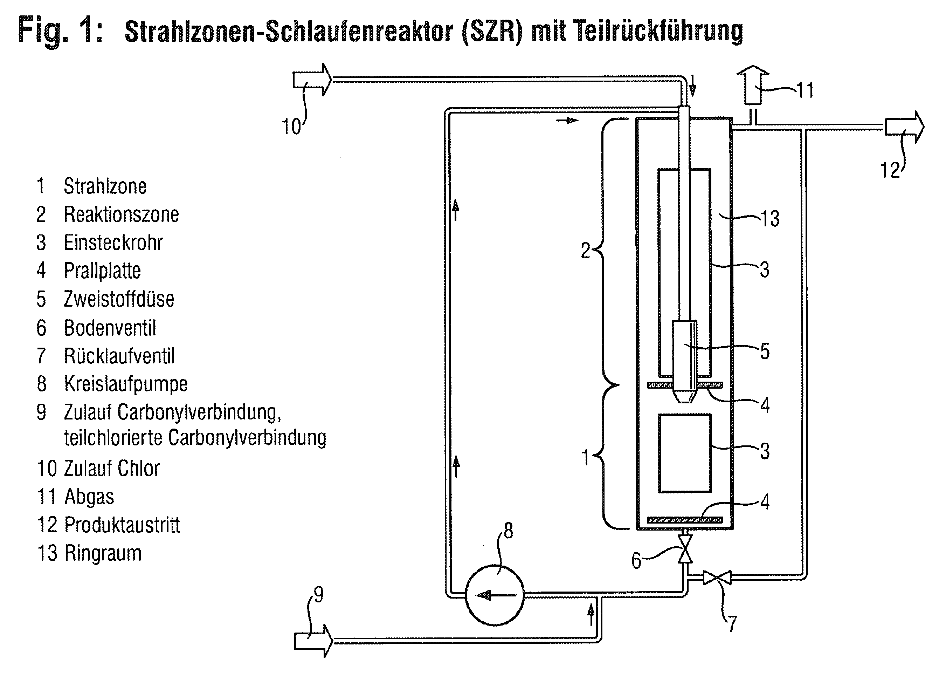

Beispielhaft soll die Funktionsweise der Jet Loop Reaktoren am Strahlzonen-Schlaufenreaktor, wie er aus DE-198 42 332 (incorporated by reference) bekannt ist, aufgezeigt werden (siehe Figur 1 Strahlzonen-Schlaufenreaktor (SZR) mit Teilrückführung).

Der Strahlzonen-Schlaufenreaktor wird in zwei verschiedene Bereiche unterteilt. Unterhalb der oberen Prallplatte 4 befindet sich die so genannte Strahlzone 1. Dieser Bereich kann als Kompaktreaktor mit geringem Höhen-Durchmesser-Verhältnis betrachtet werden. Durch die Kreislaufpumpe 8 wird über die Zweistoffdüse 5 ein Freistrahl erzeugt. Über den Zulauf Chlor 10 wird die Gasphase an der Zweistoffdüse 5 und im Freistrahl fein dispergiert. Das Gas-/Flüssigkeitsgemisch wird durch das untere Einsteckrohr 3 bis auf die untere Prallplatte 4 des Reaktors getrieben. An der unteren Prallplatte 4 erfolgt eine Umlenkung des Gemischs, was einen Aufstieg im Ringraum 13 der Strahlzone 1 nach sich zieht. Durch den Freistrahl der Zweistoffdüse 5 wird ein Teil der Flüssigkeit wieder in das Einsteckrohr 3 angesaugt, was zu der Bildung einer Schlaufenströmung führt. Der Teil des Gemischs, der nicht mehr angesaugt wird, gelangt an der oberen Prallplatte 4 vorbei in die Reaktionszone 2. Da sich im Ringraum ein höherer Gasgehalt als im Einsteckrohr einstellt, kommt es zu einer Dichtedifferenz, die eine Rückströmung verursacht. Durch diesen Effekt bildet sich eine Schlaufe nach dem Airlift-Prinzip aus. Das Produkt kann über den Produktaustritt 12 entnommen werden. Eine Produktrückführung zur Zweistoffdüse 5 ist über das Rücklaufventil 7 oder über eine Abnahme aus der Strahlzone 1 durch das Bodenventil 6 möglich.

Aufgrund der gezielt einstellbaren Parameter zur Reaktionsführung und reproduzierbarer Verfahrensbedingungen weisen diese nach dem erfindungsgemäßen Verfahren hergestellten chlorierten oder teilchlorierten Carbonylverbindungen hohe Selektivitäten auf und machen eine anschließende Isolierung des Produktes einfacher oder überflüssig. Dabei dient die fraktionierte Destillation als häufigste Isolierungsmethode.

Im Folgenden wird die Erfindung anhand von Beispielen näher erläutert.

Die nachfolgenden Beispiele geben das allgemeine Anwendungsgebiet der vorliegenden Erfindung wider und sind nicht einschränkend zu verstehen. Prozentangaben sind, soweit nicht anders angegeben, als Massenprozente zu verstehen.

Beispiele:

**Beispiel 1:**

Teilkontinuierliche Chlorierung von Monochloraceton mit Chlor zu 1,1,3 Trichloraceton nach dem erfindungsgemäßen Verfahren im Strahlzonen-Schlaufenreaktor mit Teilrückführung (siehe Figur 1).

Die Selektivität an 1,1,3 Trichloraceton ist wie folgt definiert:

$$Se(1.1.3-TCA) = \frac{1.1.3-TCA}{1.1.3-TCA+1.3-DCA+1.1-DCA+1.1.1-TCA+1.1.3.3-TeCA+1.1.1.3-TeCA}$$

TCA = Trichloraceton
DCA = Dichloraceton
TeCA = Tetrachloraceton

| Temperatur: | 40°C |
|---|---|
| Druck: | Normaldruck |
| Volumenstrom $Cl_2$: | 4-5 [$Nm^3$/h] |
| Reaktionsdauer: | 10h |
| Einsatz: | 150 kg Monochloraceton |
| Umsatz nach 10h (GC): | 99% |
| Selektivität nach 10h: | 35% 1,1,3 TCA |

Durchführung:

Monochloraceton wird über den Zulauf 9 in die Reaktionsanlage vorgelegt und auf 40°C erwärmt. Durch das Öffnen des Bodenventils 6 und die Inbetriebnahme der Kreislaufpumpe 8 wird ein Freistrahl über die Zweistoffdüse 5 erzeugt. Das Chlor wird über den Zulauf 10 in den Strahlzonen-Schlaufenreaktor über die Zweistoffdüse 5 eingebracht. Die Temperatur wird während der Chlordosierung über einen externen Kreislaufkühler auf 40°C gehalten. Der Umsatz und die Selektivität werden per Gaschromatographie verfolgt. Durch die erzielte Selektivität kann auf die Jod-Zugabe als Katalysator verzichtet werden. Die Produktisolierung erfolgt über eine Reindestillation.

**Beispiel 2:**

Kontinuierliche Chlorierung von Monochloraceton mit Chlor zu 1,1,3 Trichloraceton nach dem erfindungsgemäßen Verfahren im Strahlzonen-Schlaufenreaktor mit Teilrückführung.

Die Selektivität an 1,1,3 Trichloraceton ist wie folgt definiert:

$$Se(1.1.3-TCA) = \frac{1.1.3-TCA}{1.1.3-TCA+1.3-DCA+1.1-DCA+1.1.1-TCA+1.1.3.3-TeCA+1.1.1.3-TeCA}$$

| Temperatur: | 40°C |
|---|---|
| Druck: | Normaldruck |
| Volumenstrom $Cl_2$: | 4-5 [$Nm^3/h$] |
| Volumenstrom Monochloraceton: | 15-20 [$Nm^3/h$] |
| Reaktionsdauer: | 10h |
| Umsatz (GC): | 99% |
| Selektivität nach 10h: | 35% 1,1,3 TCA |

Durchführung:

Nach der teilkontinuierlichen Chlorierung von Monochloraceton mit Chlor nach Beispiel 1 und Inbetriebnahme der Kreislaufpumpe 8 über das Bodenventil 6 wird Monochloraceton über Zulauf 9 und Chlor über Zulauf 10 kontinuierlich über die Zweistoffdüse 5 eingebracht. Die Temperatur wird über einen externen Kreislaufkühler konstant auf 40°C gehalten. Das fertige Produkt wird kontinuierlich über den Ablauf 12 erhalten. Der Umsatz und die Selektivität werden per Gaschromatographie verfolgt. Durch die erzielte Selektivität kann auf die Jod-Zugabe als Katalysator verzichtet werden. Die Produktisolierung erfolgt über eine Reindestillation.

**Beispiel 3:**

Teilkontinuierliche Chlorierung von Malonsäure-diethylester mit Chlor zu Chlormalonsäure-diethylester nach dem erfindungsgemäßen Verfahren im Strahlzonen-Schlaufenreaktor mit Teilrückführung (siehe Figur 1).

Die Selektivität an Chlormalonsäure-diethylester (DECM) ist wie folgt definiert:

$$Se(DECM) = \frac{DECM}{DECM + DEDCM}$$

DECM = Chlormalonsäure-diethylester
DEDCM = Dichlormalonsäure-diethylester

| Temperatur: | 50°C |
|---|---|
| Druck: | Normaldruck |
| Volumenstrom $Cl_2$: | 4-5 [$Nm^3/h$] |

(fortgesetzt)

| Reaktionsdauer: | 10h |
|---|---|
| Einsatz: | 160 kg Malonsäure-diethylester |
| Umsatz nach 10h (GC): | 99% |
| Selektivität nach 10h: | 98% DECM |

Durchführung:

Analog Beispiel 1. Malonsäure-diethylester wird in die Reaktionsanlage vorgelegt und auf 50°C erwärmt. Nach der Inbetriebnahme der Kreislaufpumpe wird Chlor in den Strahlzonen-Schlaufenreaktor über die Dosierdüse eingebracht. Die Temperatur wird während der Chlordosierung über einen externen Kreislaufkühler auf 50°C gehalten. Der Umsatz und die Selektivität werden per Gaschromatographie verfolgt. Auf eine nachfolgende Produktisolierung kann auf Grund der erzielten Selektivität verzichtet werden.

**Beispiel 4:**

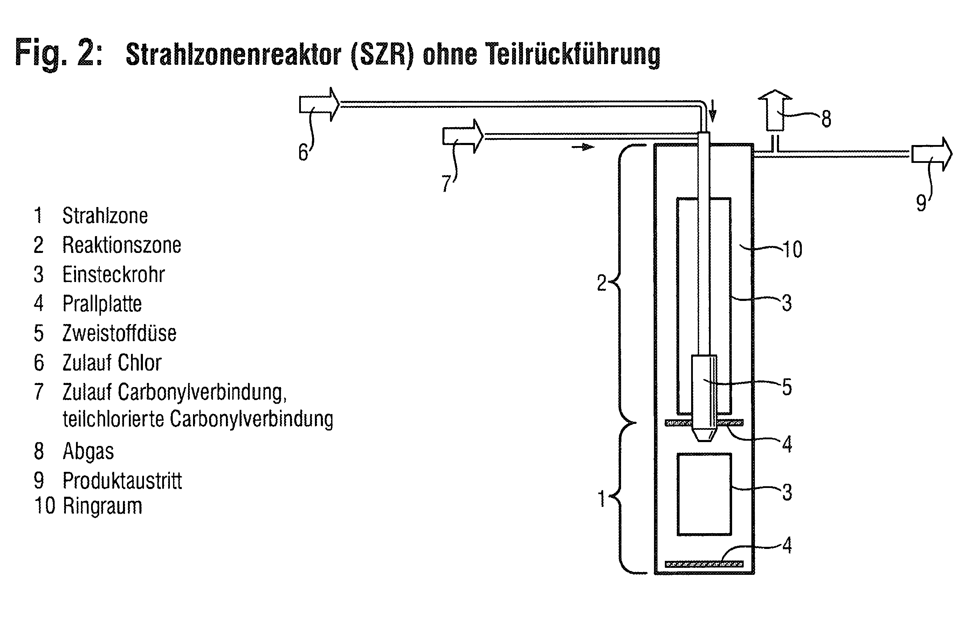

Kontinuierliche Chlorierung von Acetessigsäureethylester mit Chlor zu 2 Chlor-Acetessigsäureethylester nach dem erfindungsgemäßen Verfahren im Strahlzonen-Schlaufenreaktor ohne Teilrückführung (siehe Figur 2).
Die Selektivität an 2 Chlor Acetessigsäureethylester ist wie folgt definiert:

$$Se(2-Cl-ACE) = \frac{2-ClACE}{2-ClACE + 2.4-DClACE}$$

2-C1-ACE = 2 Chlor Acetessigsäureethylester
2,4-DC1ACE = 2,4 Dichlor Acetessigsäureethylester

| Temperatur: | 35°C |
|---|---|
| Druck: | Normaldruck |
| Volumenstrom $Cl_2$: | 40-45 [$Nm^3$/h] |
| Reaktionsdauer: | 10h |
| Massenstrom Acetessigsäureethylester: | 1800 - 2000 [kg/h] |
| Umsatz nach 10h (GC): | 10% |
| Selektivität nach 10h: | 96,5% 2 Cl-ACE |

Durchführung:

Acetessigsäureethylester wird über den Zulauf 7 kontinuierlich über die Zweistoffdüse 5 in den Strahlzonen-Schlaufenreaktor gefördert. Nach der Ausbildung des Freistrahls in der Strahlzone 1 wird Chlor kontinuierlich über Zulauf 6 und Zweistoffdüse 5 in den Strahlzonen-Schlaufenreaktor eingebracht. Die Temperatur wird durch vorgekühltes Acetessigsäureethylester auf 35°C gehalten. Das fertige Produkt wird kontinuierlich über den Ablauf 9 erhalten. Zur weiteren Isolierung wird der überschüssige Acetessigsäureethylester aus dem Reaktionsgemisch abdestilliert und der Chlorierung rückgeführt . Der Umsatz und die Selektivität werden per Gaschromatographie verfolgt.

**Beispiel 5:**

Kontinuierliche Chlorierung von 2 Acetoacetamid mit Chlor zu Chloracetoacetamid nach dem erfindungsgemäßen Verfahren im Strahlzonen-Schlaufenreaktor ohne Teilrückführung (siehe Figur 2).
Die Selektivität an Chloracetoacetamid ist wie folgt definiert:

$$Se(CAA) = \frac{CAA}{CAA + DCAA}$$

CAA = Chloracetoacetamid
DCAA = Dichloracetoacetamid

| | |
|---|---|
| Temperatur: | -5°C |
| Druck: | Normaldruck |
| Volumenstrom $Cl_2$: | 20-30 [Nm$^3$/h] |
| Reaktionsdauer: | 1h |
| Volumenstrom Acetessigsäureethylester: | 125 [Nm$^3$/h] |
| Umsatz nach 1h (GC): | 99% |
| Selektivität nach 1h: | 98,5% CAA |

Durchführung:

Analog Beispiel 4 wird Acetoacetamid über die Zweistoffdüse in den Strahlzonen-Schlaufenreaktor gefördert. Nach der Ausbildung des Freistrahls in der Strahlzone 1 wird Chlor kontinuierlich über die Zweistoffdüse in den Strahlzonen-Schlaufenreaktor eingebracht. Die Temperatur wird durch vorgekühltes Acetoacetamid und über die Mantelkühlung auf -5°C gehalten. Das Zielprodukt fällt als Feststoff aus und wird kontinuierlich über den Ablauf erhalten. Zur weiteren Isolierung wird der Feststoff über eine Zentrifuge abgetrennt. Der Umsatz und die Selektivität werden per Gaschromatographie verfolgt.

**Beispiel 6:**

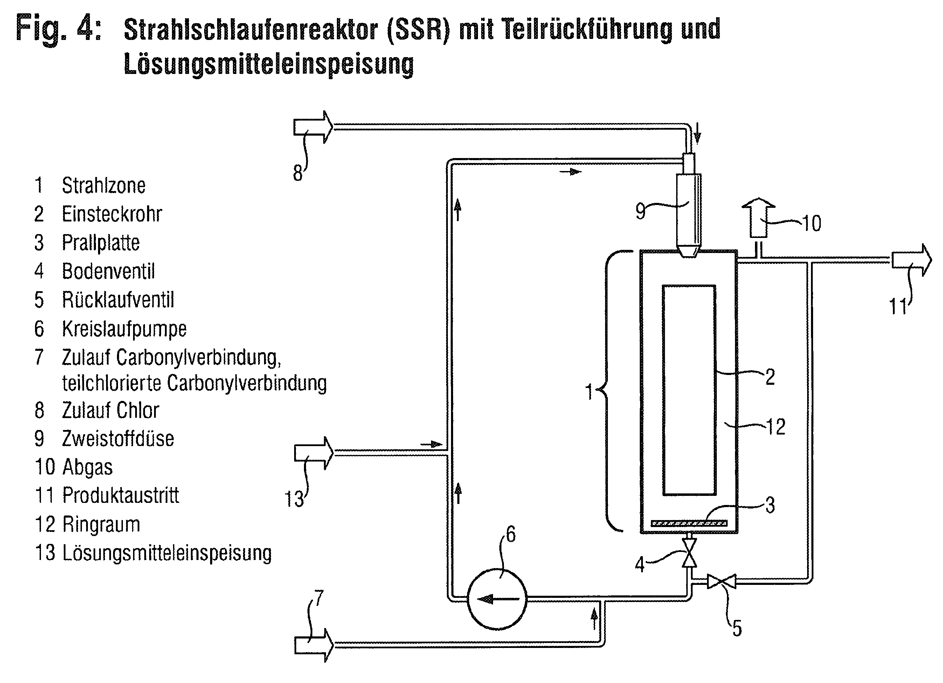

Kontinuierliche Chlorierung von Propionaldehyd in Dichlormethan mit Sulfurylchlorid zu 2-Chlorpropionaldehyd im Strahlschlaufenreaktor mit Teilrückführung (siehe Figur 4).
Die Selektivität an 2-Chlorpropionaldehyd ist wie folgt definiert:

$$Se(CPA) = \frac{CPA}{CPA + DCPA}$$

CPA = 2-Chlorpropionaldehyd
DCPA = 2,2-Dichlorpropionaldehyd

| | |
|---|---|
| Temperatur: | 25°C |
| Druck: | Normaldruck |
| Massenstrom SOCl2: | 450 - 500 [Kg/h] |
| Massenstrom Propionaldehyd: | 200 - 220 [Kg/h] |
| Massenstrom Dichlormethan: | 200 - 220 [Kg/h] |
| Reaktionsdauer: | 10 h |
| Umsatz (GC): | 99% |
| Selektivität nach 10h: | 98% CPA |

Durchführung:

Dichlormethan wird über den Zulauf 13 in die Reaktionsanlage vorgelegt. Durch das Öffnen des Bodenventils 4 und die Inbetriebnahme der Kreislaufpumpe 6 wird ein Freistrahl über die Zweistoffdüse 9 erzeugt. Propionaldehyd wird über Zulauf 7 und Sulfurylchlorid über Zulauf 8 kontinuierlich über die Zweistoffdüse 9 eingebracht. Gleichzeitig erfolgt eine kontinuierliche Zudosierung von Dichlormethan als Lösungsmittel über Zulauf 13 in den Reaktor. Die Temperatur wird über einen externen Kreislaufkühler konstant auf 25°C gehalten. Das fertige Produkt wird kontinuierlich über den Ablauf 12 erhalten. Der Umsatz und die Selektivität werden per Gaschromatographie verfolgt. Durch die erzielte Selektivität kann auf eine anschließende fraktionierte Reindestillation verzichtet werden. Zur Produktisolierung wird als Lösungsmittel Dichlormethan abdestilliert.

**Beispiel 7:**

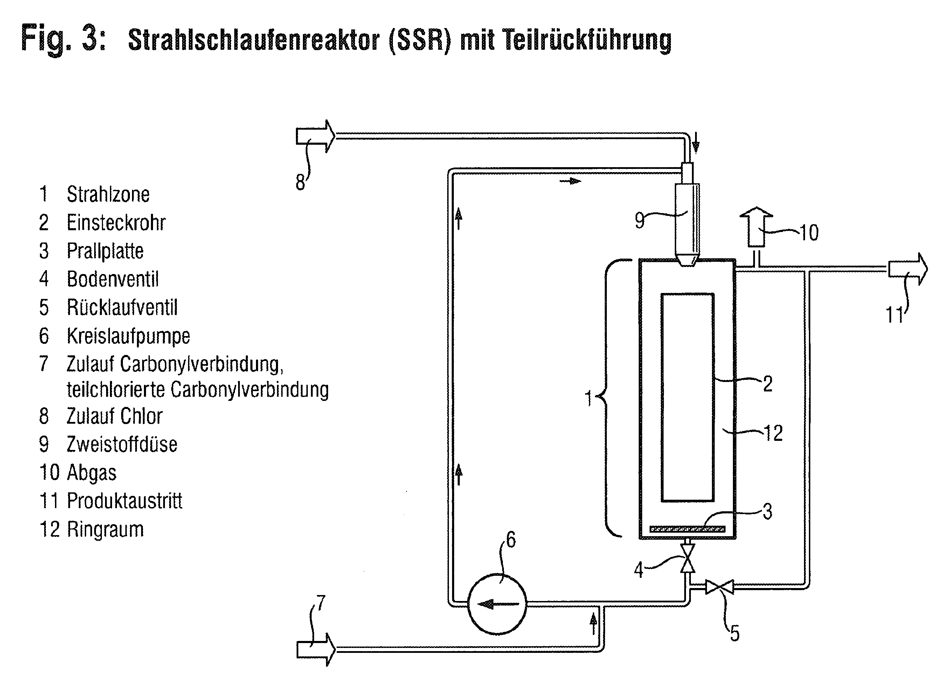

Teilkontinuierliche Chlorierung von Acetylaceton im Strahlschlaufenreaktor mit Thionylchlorid zu 3-Chlor-Acetylaceton mit Teilrückführung (siehe Figur 3).
Die Selektivität an 3-Chlor-Acetylaceton ist wie folgt definiert:

$$Se(3ClAcAc) = \frac{3ClAcAc}{3ClAcAc + 3,3ClAcAc}$$

3ClAcAc = 3-Chlor-Acetylaceton
3,3ClAcAc = 3,3 Di-Chlor-Acetylaceton

| Temperatur: | 20°C |
| --- | --- |
| Druck: | Normaldruck |
| Massenstrom Thionylchlorid: | 30 - 40 [Kg/h] |
| Reaktionsdauer: | 5h |
| Einsatz: | 160 kg Acetylaceton |
| Umsatz nach 5h (GC): | 99% |
| Selektivität nach 5h: | 98% 3-Chlor-Acetylaceton |

Durchführung:

Acetylaceton wird über den Zulauf 7 in die Reaktionsanlage vorgelegt. Durch das Öffnen des Bodenventils 4 und die Inbetriebnahme der Kreislaufpumpe 6 wird ein Freistrahl über die Zweistoffdüse 9 erzeugt. Acetylaceton wird über Zulauf 7 und Thionylchlorid über Zulauf 8 kontinuierlich über die Zweistoffdüse 9 eingebracht. Die Temperatur wird über einen externen Kreislaufkühler konstant auf 20°C gehalten. Der Umsatz und die Selektivität werden per Gaschromatographie verfolgt. Durch die erzielte Selektivität kann auf eine anschließende Reindestillation verzichtet werden.

**Beispiel 8:**

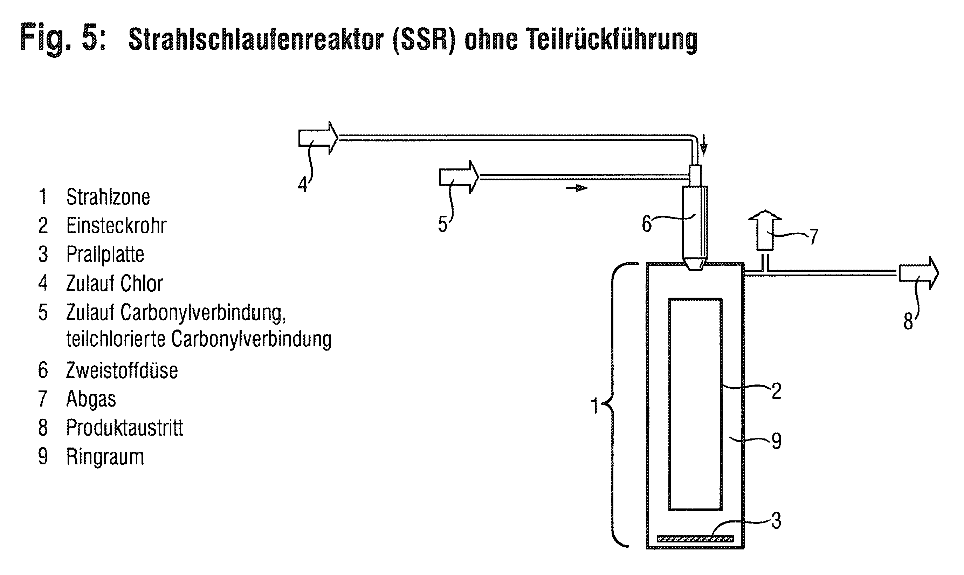

Kontinuierliche Chlorierung von Cyclopentanon mit Chlor zu 2-Chlorcyclopentanon im Strahlschlaufenreaktor ohne Teilrückführung (siehe Figur 5).
Die Selektivität an 2-Chlorcyclopentanon ist wie folgt definiert:

$$Se(CPA) = \frac{CCPA}{CCPA + 2CCPE + 3CCPE}$$

CCPA = 2-Chlorcyclopentanon
2CCPE = 3 Chlorcyclopenten 1 on
3CCPE = 3 Chlorcyclopenten 1 on

| Temperatur: | 20°C |
|---|---|
| Druck: | Normaldruck |
| Volumenstrom Chlor: | 40 - 45 [Nm$^3$/h] |
| Massenstrom Cyclopentanon: | 1800 - 2000 [Kg/h] |
| Reaktionsdauer | 12 h |
| Umsatz (GC) | 10 |
| Selektivität nach 10h: | 99% CCPA |

Durchführung:

Cyclopentanon wird über Zulauf 5 und Chlor über Zulauf 4 kontinuierlich über die Zweistoffdüse 6 eingebracht. Die Temperatur wird über die Mantelkühlung der Strahlschlaufenreaktors auf 25°C gehalten. Das fertige Produkt wird kontinuierlich über den Ablauf 8 erhalten. Der Umsatz und die Selektivität werden per Gaschromatographie verfolgt. Das überschüssige Cyclopentanon wird kontinuierlich in einer nachfolgenden Destillation vom Reaktionsprodukt getrennt und zur Chlorierung rückgeführt.

**Patentansprüche**

1. Verfahren zur Herstellung von chlorierten oder teilchlorierten Carbonylverbindungen, **dadurch gekennzeichnet, dass** unchlorierte oder teilchlorierte Carbonylverbindungen mit einem Chlorierungsmittel in einem Jet Loop Reaktor zur Reaktion gebracht werden.

2. Verfahren nach Anspruch 1, **dadurch gekennzeichnet, dass** ein Strahlzonen-Schlaufenreaktor eingesetzt wird.

3. Verfahren nach Anspruch 1 oder 2, **dadurch gekennzeichnet, dass** ein oder mehrere Chlorierungsmittel aus der Gruppe enthaltend Chlor, Sulfurylchlorid und Thionylchlorid verwendet werden.

4. Verfahren nach Anspruch 1 bis 3, **dadurch gekennzeichnet, dass** die eingesetzten unchlorierten oder teilchlorierten Carbonylverbindungen geradekettig, verzweigt oder von cyclischer Struktur sind.

5. Verfahren nach Anspruch 1 bis 4, **dadurch gekennzeichnet, dass** die Dosierung des Chlorierungsmittels zur unchlorierten oder teilchlorierten Carbonylverbindung in den Reaktor in einem Volumenstromverhältnis von 1 : 1 bis 1 : 4000 geschieht.

6. Verfahren nach Anspruch 1 bis 5, **dadurch gekennzeichnet, dass** die unchlorierten oder teilchlorierten Carbonylverbindungen in Substanz oder in Gegenwart eines oder mehrerer Lösungsmittel eingesetzt werden.

7. Verfahren nach Anspruch 1, **dadurch gekennzeichnet, dass** die Reaktion im Jet Loop Reaktor bei Temperaturen von -20°C bis 160°C stattfindet.

8. Verfahren nach Anspruch 1, **dadurch gekennzeichnet, dass** die Reaktion in Anwesenheit eines Katalysators durchgeführt wird.

9. Verfahren nach Anspruch 1 bis 8, **dadurch gekennzeichnet, dass** die Reaktion in kontinuierlicher oder halbkontinuierlicher Fahrweise stattfindet.

10. Verfahren nach Anspruch 1 bis 9, **dadurch gekennzeichnet, dass** die kontinuierliche Chlorzugabe unter Rückführung von nicht umgesetztem Chlor in den Kreislauf erfolgt.

**11.** Verfahren nach Anspruch 1 bis 10 , **dadurch gekennzeichnet, dass** die Chlorierung mit und ohne Teilrückführung erfolgt.

**Claims**

**1.** Process for preparing chlorinated or partly chlorinated carbonyl compounds, **characterized in that** unchlorinated or partly chlorinated carbonyl compounds are reacted with a chlorinating agent in a jet loop reactor.

**2.** Process according to Claim 1, **characterized in that** a jet zone loop reactor is used.

**3.** Process according to Claim 1 or 2, **characterized in that** one or more chlorinating agents from the group comprising chlorine, sulfuryl chloride and thionyl chloride are used.

**4.** Process according to Claim 1 to 3, **characterized in that** the unchlorinated or partly chlorinated carbonyl compounds used are straight-chain, branched or have a cyclic structure.

**5.** Process according to Claim 1 to 4, **characterized in that** the chlorinating agent is metered into the unchlorinated or partly chlorinated carbonyl compound in the reactor in a volume flow ratio of from 1:1 to 1:4000.

**6.** Process according to Claim 1 to 5, **characterized in that** the unchlorinated or partly chlorinated carbonyl compounds are used in substance or in the presence of one or more solvents.

**7.** Process according to Claim 1, **characterized in that** the reaction in the jet loop reactor takes place at temperatures of from -20°C to 160°C.

**8.** Process according to Claim 1, **characterized in that** the reaction is performed in the presence of a catalyst.

**9.** Process according to Claim 1 to 8, **characterized in that** the reaction takes place in continuous or semicontinuous mode.

**10.** Process according to Claim 1 to 9, **characterized in that** the continuous addition of chlorine is effected with recycling of unconverted chlorine into the circuit.

**11.** Process according to Claim 1 to 10, **characterized in that** the chlorination is effected with and without partial recycling.

**Revendications**

**1.** Procédé de fabrication de composés de carbonyle chlorés ou partiellement chlorés, **caractérisé en ce que** des composés de carbonyle non chlorés ou partiellement chlorés sont mis en réaction avec un agent de chloration dans un réacteur Jet Loop.

**2.** Procédé selon la revendication 1, **caractérisé en ce qu'**un réacteur à zone de jet et à boucle de circulation est utilisé.

**3.** Procédé selon la revendication 1 ou 2, **caractérisé en ce qu'**un ou plusieurs agents de chloration du groupe contenant le chlore, le chlorure de sulfuryle et le chlorure de thionyle sont utilisés.

**4.** Procédé selon les revendications 1 à 3, **caractérisé en ce que** les composés de carbonyle non chlorés ou partiellement chlorés utilisés sont linéaires, ramifiés ou de structure cyclique.

**5.** Procédé selon les revendications 1 à 4, **caractérisé en ce que** l'ajout de l'agent de chloration au composé de carbonyle non chloré ou partiellement chloré dans le réacteur a lieu avec un rapport entre les débits volumiques de 1:1 à 1:4 000.

**6.** Procédé selon les revendications 1 à 5, **caractérisé en ce que** les composés de carbonyle non chlorés ou partiellement chlorés sont utilisés en substance ou en présence d'un ou de plusieurs solvants.

**7.** Procédé selon la revendication 1, **caractérisé en ce que** la réaction a lieu dans le réacteur Jet Loop à des températures de -20 °C à 160 °C.

**8.** Procédé selon la revendication 1, **caractérisé en ce que** la réaction est réalisée en présence d'un catalyseur.

**9.** Procédé selon les revendications 1 à 8, **caractérisé en ce que** la réaction a lieu selon un procédé continu ou semi-continu.

**10.** Procédé selon les revendications 1 à 9, **caractérisé en ce que** l'ajout continu de chlore a lieu par recyclage du chlore non réagi dans le circuit.

**11.** Procédé selon les revendications 1 à 10, **caractérisé en ce que** la chloration a lieu avec et sans recyclage partiel.

# Fig. 1: Strahlzonen-Schlaufenreaktor (SZR) mit Teilrückführung

1 Strahlzone

2 Reaktionszone

3 Einsteckrohr

4 Prallplatte

5 Zweistoffdüse

6 Bodenventil

7 Rücklaufventil

8 Kreislaufpumpe

9 Zulauf Carbonylverbindung, teilchlorierte Carbonylverbindung

10 Zulauf Chlor

11 Abgas

12 Produktaustritt

13 Ringraum

EP 1 923 379 B1

# Fig. 2: Strahlzonenreaktor (SZR) ohne Teilrückführung

1 Strahlzone
2 Reaktionszone
3 Einsteckrohr
4 Prallplatte
5 Zweistoffdüse
6 Zulauf Chlor
7 Zulauf Carbonylverbindung,
  teilchlorierte Carbonylverbindung
8 Abgas
9 Produktaustritt
10 Ringraum

EP 1 923 379 B1

# Fig. 3: Strahlschlaufenreaktor (SSR) mit Teilrückführung

1  Strahlzone
2  Einsteckrohr
3  Prallplatte
4  Bodenventil
5  Rücklaufventil
6  Kreislaufpumpe
7  Zulauf Carbonylverbindung,
   teilchlorierte Carbonylverbindung
8  Zulauf Chlor
9  Zweistoffdüse
10 Abgas
11 Produktaustritt
12 Ringraum

EP 1 923 379 B1

# Fig. 4: Strahlschlaufenreaktor (SSR) mit Teilrückführung und Lösungsmitteleinspeisung

1 Strahlzone

2 Einsteckrohr

3 Prallplatte

4 Bodenventil

5 Rücklaufventil

6 Kreislaufpumpe

7 Zulauf Carbonylverbindung, teilchlorierte Carbonylverbindung

8 Zulauf Chlor

9 Zweistoffdüse

10 Abgas

11 Produktaustritt

12 Ringraum

13 Lösungsmitteleinspeisung

EP 1 923 379 B1

## Fig. 5: Strahlschlaufenreaktor (SSR) ohne Teilrückführung

1 Strahlzone
2 Einsteckrohr
3 Prallplatte
4 Zulauf Chlor
5 Zulauf Carbonylverbindung,
  teilchlorierte Carbonylverbindung
6 Zweistoffdüse
7 Abgas
8 Produktaustritt
9 Ringraum

EP 1 923 379 B1

**IN DER BESCHREIBUNG AUFGEFÜHRTE DOKUMENTE**

**In der Beschreibung aufgeführte Patentdokumente**

- EP 0234503 A
- GB 2036016 A
- DE 19842332
- DE 10010594

**In der Beschreibung aufgeführte Nicht-Patentliteratur**

- **Van Dierendonck.** *Industrial Engineering Chemistry Research,* 1998, vol. 37, 734-738
- Bubble Columns 4. Jet Loop Reactors. **ZEHNER, P.** Ullmann's Encyclopedia of Industrial Chemistry. 15. Juni 2000